Europäisches Patentamt

European Patent Office

Office européen des brevets

(11) Publication number: **0 177 167**
A1

# EUROPEAN PATENT APPLICATION

(21) Application number: 85306080.4

(22) Date of filing: 28.08.85

(51) Int. Cl.⁴: **B 01 D 13/04**
B 29 C 53/58, D 04 H 3/07

(30) Priority: 06.09.84 GB 8422530

(43) Date of publication of application:
09.04.86 Bulletin 86/15

(84) Designated Contracting States:
AT BE CH DE FR GB IT LI LU NL SE

(71) Applicant: The Shirley Institute

Didsbury Manchester M2O 8RX(GB)

(72) Inventor: Floyd, Kenneth Lewthwaite
9 Upton Avenue
Cheadle Hulme Cheadle Cheshire(GB)

(72) Inventor: Katona, Josef
815 Wilmslow Road
Didsbury Manchester(GB)

(74) Representative: Lawrence, John Gordon et al,
McNeight & Lawrence Regent House Heaton Lane
Stockport, Cheshire SK4 1BS(GB)

(54) Porous tubes.

(57) Porous tubes are made from thermally bonded non-woven. Such tubes can be made seamless and suitable for lining with a semi-permeable membrane for reverse osmosis filtration. Fleece or card webs (32) can be wrapped around a mandrel (35) and compacted as by rolling and/or needling and thermobonded on the mandrel which may be tapered to ease removal.

FIG.3

EP 0 177 167 A1

Croydon Printing Company Ltd.

0177167

## POROUS TUBES

This invention relates to porous tubes and to methods and apparatus for making them.

Porous tubes according to the invention may be suitable for lining with a semi-permeable membrane to act as a support for the same in apparatus for filtering by reverse osmosis.

Typically, such tubes have an internal diameter of 1.5 cm or so and lengths up to about 4 metres. Conventionally, they are made by wrapping a tape of suitable thermoplastic material helically on to a mandrel and forming welded seams between adjacent turns of the helix. Such seams become non-porous and reduce the effective surface area of the support.

Porous tubes can also be made by a needling process. However, such tubes, especially when they have relatively thin walls, tend to be too flexible for some applications.

The present invention provides an alternative

porous tube and a method and apparatus for making the same which have advantages over previous techniques.

The invention comprises a porous tube comprising a wall material of thermally bonded nonwoven.

The tube may be seamless, and is suitable for lining with a semi-permeable membrane.

The invention also comprises a method of making a porous tube comprising forming a tubular fibre mass, including a melt component and a structure component, around a mandrel, and compacting the fibre mass and heating and cooling it to cause said melt component to melt and reset to form bonds between structure component fibres, and removing the resultant thermobonded tube from the mandrel.

The web or fleece may be wrapped, at approximately final tube length, several times about the mandrel, to form a single length of tube - which will be seamless. The web or fleece may be wrapped with a small helix angle to form a tube with tapered ends.

The mandrel itself may be slightly tapered to facilitate removal of the finished tube therefrom.

The wrappea web or fleece may be compacted by an arrangement of rotary rollers extending the length of the mandrel. The roller arrangement may be located in an oven to effect the melting of the melt component.

The tube may be formed continously by wrapping a fibre web or fleece continuously on one end of a mandrel and moving the wrapped web or fleece along the mandrel whilst compacting it. This mandrel may be tapered, the tube being continuously moved towards the narrow end of the manarel.

The tubular fibre mass may be needle punched while on the mandrel. The mandrel may have holes in it into which needles can pass from a guide member adjacent the manarel with alignea apertures, needle oscillating means ariving the needles through the fibre mass between the guiae member ana the mandrel.

The guide means may be cylindrically disposed about the mandrel, the needles oscillating radially.

The web or fleece may be supplied from a carding process. A card web may be folded, as by a cross-folaer, to increase its thickness. The web or fleece may have portions of aifferent materials whereby aifferent parts of the tube will be of such aifferent

materials. A first-wrapped part of the web may be of a first material and a second-wrapped part of a second material whereby the inside of the tube is of the first material and the outside of the tube of the second material.

The invention also comprises apparatus for making a porous tube comprising a mandrel on which a web or fleece of fibre can be wrapped to form a tubular fibre mass, compacting means for compacting said mass while on said mandrel, and heating means for thermobonding said mass.

Said compacting means may comprise an arrangement of rollers parallel to and surrounding said mandrel in which the mandrel with its wrapped fibre mass can be rolled.

The invention will be further apparent from the following description, with reference to the figures of the accompanying drawing, which illustrate, by way of example only, certain features of one method embodying the invention.

Of the drawings:-

Figure 1   shows one embodiment of apparatus for the formation of a tube from a fibrous mass;

Figure 2   shows apparatus for subjecting the tube to mechanical and thermal treatment.

Figure 3   is a diagrammatic illustration of a continuous operating arrangement, and

Figure 4   is a cross-section through a needling arrangement.

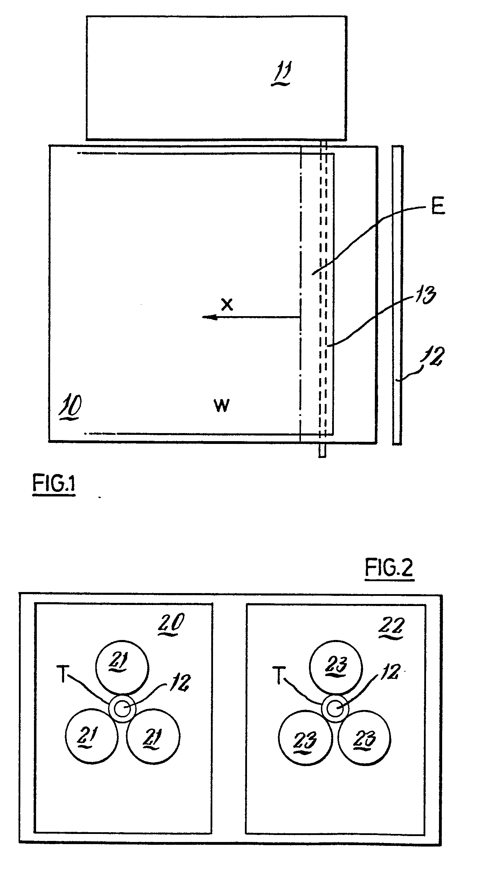

Referring first to Figure 1, it will be seen that a fibrous web W is delivered to a platform 10 from a carding engine 11 which may have an associated cross-folder. The web includes thermoplastic material (the melt component) having a lower melting point than the remainder of the material therein (the structure component), and may conveniently be produced from a bi-component fibre, though blends of different fibre types are also possible.

A mandrel 12 having a length corresponding to the length of tube T to be produced extends alongside one edge of the platform 10. When the web W has been

delivered sufficiently to extend over the length of the platform 10, the mandrel 12 is laid onto the web W at the position indicated by the chain dotted line and the edge of the web E is folded by bar member 13 over the mandrel 12. The mandrel 12 is then rolled across the platform 10 in the direction of the arrow X to cause the web to be wrapped around the mandrel 12 to form a tube T.

A valuable feature is the ability to supply fibres to the carding engine 11 such that one half of the web is of different type from the other enabling the inside of the tube T to be of different material, such as predominantly polypropylene (which readily accepts polysulphone to form a semi-permeable membrane lining), from the outside which may be predominantly of polyester (which gives dimensional stability).

The mandrel 12, which has a slight taper to facilitate tube removal, is then transferred to an oven 20 shown diagrammatically in Figure 2.

Rollers 21 are closed to grip the tube T against the mandrel 12 and rotated to effect mechanical compaction of the fibrous mass as it is heated to soften the thermoplastic component. The mandrel 12 is then transferred to a cooling zone 22 where further rollers 23 are closed to grip the tube T and rotated to continue compaction as the tube T cools to complete the thermobonaing of the fibrous structure.

The manarel 12 is then removea from the zone 22 and the completea tube T slia therefrom.

If, in rolling the manarel 12 across the platform 10 the airection is alterea slightly to introauce a small aegree of helical wrapping of the web E, the tube will have tapered ends.

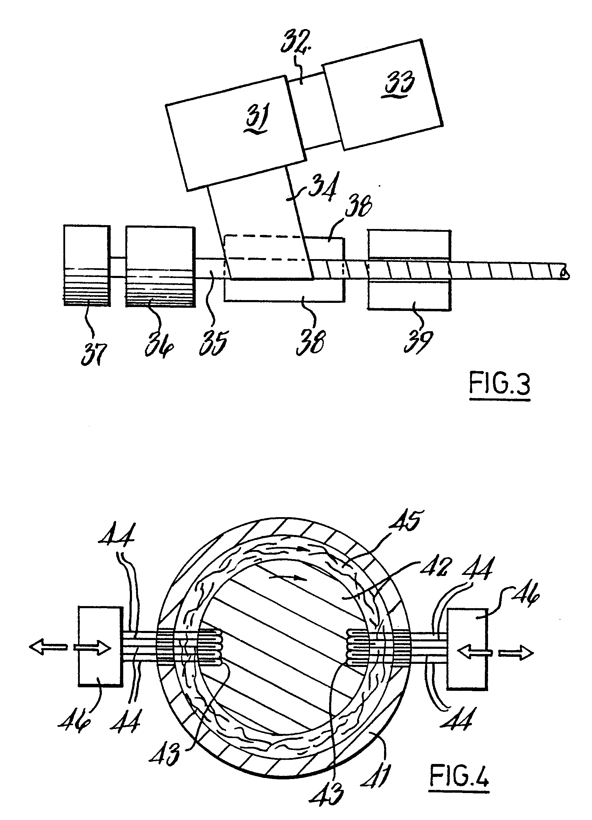

Figure 3 shows a continuously operating arrangement in which a carding machine 31 delivers a web 32 to a cross-folaer 33 which in turn delivers a multi-ply fleece 34 to a continuously rotating mandrel 35 which is cantileverea from a bearing 36 ana driving arrangement 37. Roller means 38 engage the fleece 34 to compact it on ana move it along the mandrel 35 away from the bearing 36. Still on the mandrel 35 the wrapped and compactea fleece 34 is heated in an oven 39, which may comprise radiant heaters, and then moves off the end of the mandrel 35.

Figure 4 shows in cross-section a needling arrangement which can be used with the apparatus of Figure 1 or Figure 3 comprising guide means 41 arranged cylindrically around the rotating mandrel 42 which has holes 43 into which needles 44 can pass. The needles 44 are reciprocated through the fibre mass 45 between the guide means 41 and the mandrel 42 by driven needle bars 46.

The intensity of the needling can be very much less than that required for a purely needled tube, which means that the density of the tube wall can be very much less for a given strength. The rigidity of the tube can also be controlled by the amount of needling effected and/or by the amount and nature of thermobonding effected.

Porous tubes made as described can be used, as already remarked, in reverse osmosis filtration apparatus. Polyester tubes can be manufactured also for medical and hygiene purposes, for example for prosthetic arteries, tampon sleeves and so on.

## CLAIMS

1.      A porous tube comprising a wall material of thermally bonded non-woven fibres, the thermal bonding agent being part of the web or fleece from which the tube is made.

2.      A tube according to claim 1, being seamless.

3.      A tube according to claim 1 or claim 2, having an internal diameter between 0.5 and 5 cm.

4.      A tube according to any one of claims 1 to 3, having a length of up to 4 metres.

5.      A tube according to any one of claims 1 to 4, lined with a semi-permeable membrane.

6.      A method of making a porous tube comprising forming a tubular fibre mass, including a melt component and a structure component, around a mandrel, and compacting the fibre mass and heating and cooling it to cause said melt component to melt and reset to form bonds between structure component fibres, and removing the resultant thermobonded tube from the mandrel.

7. A method of making a porous tube according to claim 6 in which the melt component is one or more of polyethylene, polypropylene or other polyolefine or polyamioe ano the structure component is polyester fibre.

8. A methoo accoroing to claim 6 or claim 7, in which the tube is formeo without a seam.

9. A methoo according to claim 8, in which a fibrous web or fleece is wrapped, at approximately tube length, several times about the mandrel, to form a single length of tube.

10. A method according to claim 9, in which the fibrous web or fleece is wrapped with a helix angle to form a tube with tapered ends.

11. A methoo accoroing to any one of claims 7 to 10, in which the mandrel is slightly tapered to facilitate removal of the finisheo tube therefrom.

12. A method according to any one of claims 9 to 11, in which the wrappeo web or fleece is compacted by an arrangement of rotary rollers extending the length of the mandrel.

13.    A method according to claim 12, in which the roller arrangement is located in an oven.

14.    A method according to claim 13 in which the rollers are heated.

15.    A method according to any one of claims 6 to 9, in which the tube is formed continuously by wrapping a fibre web or fleece continously on one end of a mandrel and moving the wrapped fleece along the mandrel whilst compacting it and heating it to form the bonding.

16.    A method according to claim 15, in which the mandrel is tapered and the tube is continuously moved towards the narrow end of the mandrel.

17.    A method according to any one of claims 7 to 16, in which the tubular fibre mass is needle punched while on the mandrel.

18.    A method according to claim 17, in which the mandrel has holes into which needles can pass and there is a guide member adjacent the mandrel with aligned apertures, and needle oscillating means drive the needles through the fibre mass between the guide member and the mandrel.

19.    A methoo accoraing to claim 18, saia guide means being cylinarically aisposea about the manarel and the neeales oscillating raaially.

20.    A methoo accoraing to any one of claims 7 to 19, in which the web or fleece is suppliea from a caraing process.

21.    A methoa accoraing to any one of claims 7 to 20, in which the web or fleece has portions of different materials whereby aifferent parts of the tube will be of such aifferent materials.

22.    A method according to claim 21, in which a first-wrappea part ot the web is of a first material ana a second-wrapped part of a second material whereby the insiae of the tube is of the first material and the outsiae of the secona material.

23.    A methoa accoraing to claim 22 wherein said first and secona materials are polyolefine or polyamide ana a polyester respectively.

24.    Apparatus for making a porous tube comprising a mandrel on which a web or fleece of fibre can be wrapped to form a tubular fibre mass, compacting means for

compacting saia mass while on saia manarel ana heating means for thermobonaing saia mass.

25.     Apparatus according to claim 24, saia mandrel being taperea for ease of removal of saia thermobonded fibre mass.

26.     Apparatus accoraing to claim 24 or claim 25, saia compacting means comprising an arrangement of roller parallel to ana surrounding said manarel in which the manarel with its wrappea fibre mass can be rolled.

27.     Apparatus according to any one of claims 24 to 26, saia compacting means comprising a needling arrangement.

28.     Apparatus accoraing to any one of claims 24 to 27, said heating means comprising an oven housing said manarel.

29.     A tube maae by any of the above means coated with a semi-permeable or porous membrane for use in reverse osmosis, ultrafiltration or microfiltration.

1/2

0177167

FIG.1

FIG.2

FIG.3

FIG.4

European Patent
Office

**EUROPEAN SEARCH REPORT**

0177167

Application number

EP 85 30 6080

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE-A-2 035 371 (UNITED KINGDOM ATOMIC ENERGY AUTHORITY)<br><br>* page 2, lines 1-12; page 3, lines 15-32; page 8, lines 7-10; example 1; figures 1,2 * | 1-3,5-8,15,22,24,29 | B 01 D 13/04<br>B 29 C 53/58<br>D 04 H 3/07 |
| A | DE-A-2 035 119 (UNITED KINGDOM ATOMIC ENERGY AUTHORITY)<br>* page 2, lines 1-30; page 3, line 25 - page 4, line 9; example (page 13) * | 1,2,5-8 | |
| A | DE-A-2 006 323 (ALLIED CHEMICAL CORP.)<br>* claims 1,2,4,7 * | 1,6,7 | |
| A | US-A-3 933 557 (D.B. PALL)<br><br>* column 11, line 41 - column 12, line 11 * | 6-8,11,12,15,16 | TECHNICAL FIELDS SEARCHED (Int. Cl.4)<br><br>B 01 D 13/04<br>B 29 C 53/56<br>B 29 C 53/58<br>D 04 H 1/46<br>D 04 H 3/07 |
| A | DE-B-2 652 537 (RONTEX AMERICA, INC.)<br>* column 5, lines 11-37; figures 1,3 * | 12,15,17-20 | |
| A | US-A-4 214 612 (W.J. DE PUTTER)<br><br>* column 3, lines 11-14, 33-35; figures 1,3 * | | |

--- -/-

The present search report has been drawn up for all claims

| Place of search | Date of completion of the search | Examiner |
|---|---|---|
| BERLIN | 21-11-1985 | BRUCK |

European Patent
Office

**EUROPEAN SEARCH REPORT**

01 77 167 Application number

EP 85 30 6080

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 081 869  (WAFILIN B.V.)  * page 6, line 27 - page 7, line 15; figures 1,2 *  ----- | | |

TECHNICAL FIELDS
SEARCHED (Int. Cl.4)

The present search report has been drawn up for all claims

| Place of search | Date of completion of the search | Examiner |
|---|---|---|
| BERLIN | 21-11-1985 | BRUCK |

EPO Form 1503 03 82